# EUROPEAN PATENT SPECIFICATION

(11) **EP 2 231 855 B1**
(45) Date of publication and mention of the grant of the patent: **07.10.2015**
(21) Application number: 08847410.1
(22) Date of filing: 05.11.2008
(51) Int. Cl.: C12N 1/16, C12N 1/32, C11C 3/00

(54) **A NEW STRAIN OF YARROWIA LIPOLYTICA AND ITS USE IN THE INDUSTRIAL RECLAMATION OF GLYCEROL FRACTIONS OBTAINED DURING BIODIESEL PRODUCTION**
NEUER YARROWIA LIPOLYTICA-STAMM UND DESSEN VERWENDUNG BEI DER GROSSTECHNISCHEN RÜCKGEWINNUNG VON WÄHREND DER BIODIESELHERSTELLUNG ERHALTENEN GLYCERINFRAKTIONEN
NOUVELLE SOUCHE DE YARROWIA LIPOLYTICA ET SON UTILISATION DANS LA RÉUTILISATION INDUSTRIELLE DE FRACTIONS DE GLYCÉROL OBTENUES AU COURS DE LA PRODUCTION DE BIODIESEL

(30) Priority: 05.11.2007 PL 38368507
(43) Date of publication of application: 29.09.2010
(73) Proprietor: Skotan Spolka Akcyjna, 41-506 Chorzów (PL)
(72) Inventor: RYMOWICZ, Waldemar, PL-50-375 Wroclaw (PL)
(74) Representative: Witek, Rafal
(86) International application number: PCT/PL2008/000077
(87) International publication number: WO 2009/061225

(56) References cited:
- WO-A-2007/017356
- PAPANIKOLAOU SERAPHIM ET AL: "Lipid production by Yarrowia lipolytica growing on industrial glycerol in a single-stage continuous culture" BIORESOURCE TECHNOLOGY, ELSEVIER, GB, vol. 82, no. 1, 1 March 2002 (2002-03-01), pages 43-49, XP002429335 ISSN: 0960-8524
- PAPANIKOLAOU S ET AL: "Yarrowia lipolytica as a potential producer of citric acid from raw glycerol" JOURNAL OF APPLIED MICROBIOLOGY, OXFORD, GB, vol. 92, 1 January 2002 (2002-01-01), pages 737-744, XP007904668 ISSN: 1364-5072
- IMANDI ET AL: "Optimization of medium constituents for the production of citric acid from byproduct glycerol using Doehlert experimental design" ENZYME AND MICROBIAL TECHNOLOGY, STONEHAM, MA, US, vol. 40, no. 5, 8 March 2007 (2007-03-08), pages 1367-1372, XP005918063 ISSN: 0141-0229
- JIE-WAN KIM ET AL: "High Cell Density Culture of Yarrowia lipolytica Using a One-Step Feeding Process" BIOTECHNOLOGY PROGRESS, AMERICAN INSTITUTE OF CHEMICAL ENGINEERS, US, vol. 16, no. 4, 7 June 2000 (2000-06-07), pages 657-660, XP007904669 ISSN: 8756-7938

## Description

The subject of the present invention is an industrial method of reprocessing the glycerol fraction obtained during the production of biodiesel as well as a novel strain of *Yarrowia lipolytica* particularly suited for use in this process.

The production of reusable fuel oil components, the so called biodiesels, chiefly consists of producing fatty acid esters from natural triglycerides (usu. plant lipids) via transestrification. US Patent 2.271.619 reveals a method of transforming glycerides of higher fatty acids into esters of short alcohols through the addition of a saturated monohydroxyl aliphatic alcohol of less than five carbons in the presence of an essentially anhydrous alkali metal hydroxide as a catalyst. According to said patent the process should take place in a reactor at a temperature of 86 to 212°F (30 to 100°C). The amount of alcohol should not exceed 1.75 equivalents of glyceride, and the catalyst should be from 0.1 to 0.5% by mass of glyceride.

Subsequent patents amend or supplement this patent. US Patents 2.360.844; 2.383.632; 2.383.580; 2.383.581; 2.383.614; 2.383.633; 2.383.596; and 2.383.599 respectively describe the consecutive variants of the method described in US 2.271.619 consisting of: a) addition of the acid into the process and spray-drying; b) addition of a distillation step of the unreacted alcohol; c) evaluation of the catalyst activity, suggested pH of 5 to 7; d) application of a partial fatty acid ester technique; e) reclamation of unreacted alcohol and acidification of the liquid in order to improve the separation of esters and glycerol transformation, using various methods, of partially reacted glycerides; g) supplementation of the monohydroxyl aliphatic alcohol (not methanol) with a portion of methanol in order to improve the separation of liquid phases; as well as h) addition of a solvent in order to improve phase separation.

Other patents propose more extended modifications and improvements. US patents 2.494.366; 2.383.601; 3.963.699; 4.303.590; 4.371.470; 4.668.439; 5.399.731; 5.434.279; and 5.525.126 also largely build on the technology described in US 2.271.619. They respectively relate to: a) addition of an appropriate amount of acidic catalyst to the alkaline catalyst; b) further addition acidic estrification catalyst; c) conducting the process under constant temperature and pressure conditions, from vacuum to atmospheric pressure, d) addition of a second alkaline catalysis stage; e) addition of a second esterification stage and removal of the alkyl ester with an absorbent; f) introduction of an alcohol in vapour form; g) conducting the reaction at a lower temperature with an increased amount of acid; g) introduction of an improved method of phase separation using acid; as well as h) use of a catalyst being a mixture of calcium acetate and barium acetate.

Regardless of the technology used, the chief by-product formed through the transestrification of triglycerides in need of reclamation is the so-called glyceride fraction consisting of soluble, hydrophylic products of the reaction, meaning glycerol, and residues of the unused catalyst, fatty acid esters and other reagents used in further stages of separation of the transestrification process, such as phosphoric acid and inorganic salts.

Reclamation and/or recycling of the glycerol fraction constitute a significant problem in the production of biodiesel.

Attempts have been published in e.g., Papanikolaou et al, Bioresource Technology 2002; 82: 43-49.

The goal of the present invention is to deliver a method of easily reclaiming the glycerine fraction arising during biodiesel production. A particular goal of the present invention is to deliver an efficient method of industrially transforming the glycerol fraction into easily absorbed biomass. This method could be used on an industrial scale to reclaim glycerol fractions, taking into account the varying composition of said fraction depending on the biodiesel manufacturing process used. The biomass produced should be characterised by a high content of easily absorbable protein, vitamins and should be capable of being used as a feed additive.

Unexpectedly, such defined problems have been solved in the present invention.

The goal of the present invention is an industrial method of utilizing a glycerol fraction resulting from biodiesel characterised in that the yeast *Yarrowia lipolytica* described below is cultured on a medium consisting of an aqueous solution of 20.0 to 70.0 g/l of the glycerol fraction, from 8.5 to 15.0 g/l (NH₄)₂SO₄. from 1.5 to 6.5 g/l urea, from 0.5 to 3.0 g/l MgSO₄ x 7H₂O, from 0.1 to 2.0 g/l KH₂PO₄, from 0.1 to 2.0 g/l yeast extract, at a temperature of 25 - 35°C, and aeration at 0.2-4 L air/1L medium/minute maintaining a pH between 2.5 and 7.5. to a substantial exhaustion of the glycerol in the medium, where the culture is maintained in a periodic manner, replacing a portion of the post-culture broth at the end of each cycle with fresh medium.

The method according to the present invention is meant for use on an industrial scale, wherein the culture is maintained in a volume of at least 1000 litres. Preferentially, a separated post-culture broth contains at from 15 to 35 g/L of yeast dry mass. The production of dry mass occurs at a rate from 1.5 to 3.0 g/Lh, and the biomass production preferentially occurs at maximum efficiency from 0.4 to 0.5 g dry biomass/g glycerol fraction in the medium. The protein content of the dry biomass is from 30 to 50 % by mass. The culture makes use of the *Yarrowia lipolytica SKOTAN* strain deposited in the IBPRS under the accession number KKP 2018 p.

The SKOTAN strain of *Yarrowia lipolytica* has been deposited at a bank acting in accordance with the treaty of Budapest, and maintained by the Institute of Biotechnology of the Food and Agriculture Industry (henceforth IBPRS), ul. Rakowiecka 36. 02-532 Warsaw and was given the accession number KKP 2018 p. This is a wild-type strain, selected from among many other strains of this species tested in the course of designing the present invention, belonging to the collection of the Wroclaw University of Environmental and Life Sciences. The selection criteria consisted mainly of culture conditions based on the glycerol fraction. First of all, this strain, out of all of the strains of *Yarrowia lipolytica* tested, yielded an exceptionally preferable efficiency of biomass production and a great tolerance for deleterious culture conditions such as increasing osmotic pressure and the relatively low pH of the medium. Due to this, culturing such a strain is much simpler, since the danger of its contamination by other microorganisms is much lower. At the same time, the biomass produced possesses preferable nutritional properties such as a high content of easily absorbable protein and vitamins, particularly vitamin B. Due to this, it may be used as a high-quality feed additive.

The next subject of the present invention is the SKOTAN strain of *Yarrowia lipolytica SKOTAN* deposited in the IBPRS under the accession number KKP 2018 p.

The subject of the present invention is also the use of the SKOTAN strain of *Yarrowia lipolytica SKOTAN* deposited in the IBPRS under the accession number KKP 2018 p in the reclamation of waste arising from biodiesel production. Preferentially, the biomass produced is used in the manufacture of feeds.

### Example. 1

The composition of a medium (Medium 1) in the production of biomass of the yeast *Yarrowia lipolytica* on a medium based on a glycerol fraction from biodiesel production consists of (in g/litre):

| | |
|---|---|
| glycerol fraction | 20.0 to 70.0. preferentially about 50.0 |
| (NH₄)₂SO₄ - | 8.5 - 15.0. preferentially about 12.6 |
| Urea- | 1.5 - 6.5. preferentially about 4.0 |
| MgSO₄ x 7H₂O - | 0.5 - 3.0. preferentially about 1.0 |
| KH₂PO₄ - | 0.1 - 2.0. preferentially about 0.5 |
| Yeast extract - | 0.1 - 2.0. preferentially about 0.5 |
| Tap water - to 1000 ml | |
| pH - | 2.5 - 7.5. preferentially about 3.5 - 4.0 |

In the proportions outlined above, the components of Medium 1 should be weighed out to 1100 L and brought to 1000 L with tap water.

Following the complete dissolution of the medium components, they are transferred into a bioreactor. The medium should be supplemented with 100 L of yeast cells pre-cultured in a bioreactor with a working volume of 150 L, cultured in a medium as above.

Culture conditions for various strains of *Yarrowia lipolytica* are:
a temperature of 25 - 35°C (preferentially about 30°C ± 1), with agitation at 400 - 1200 RPM (preferentially about 700-800), aeration at 0.2-4 L air/L medium/minute (preferentially about 1-1.5 L air/L medium/minute). The pH should be automatically maintained using 10 N NaOH. Should excessive foaming occur, a defoaming agent such as ACEPOL or another should be used.

The culture should be maintained until exhaustion of glycerol in the culture medium. 200 L of the cell suspension should be retained, into which the components of (Medium 1) should be added at a mass sufficient for 1100 L, and 900 L water should be added. This culture method (periodic, cyclical) may be repeated from 5 to 15 times.

This yeast propagation protocol yields 15 - 35 g/L (preferentially about 33 g/L), of dry yeast mass at a rate of 1.5 - 3.0 g/Lh (preferentially about 2.5 g/Lh), at a n efficiency of at least 0.4 - 0.5 g dry yeast mass/g glycerol fraction, (preferentially about 0.60 g/g in the case of SKOTAN *Yarrowia lipolytica*).

The protein content of the dry yeast should be 30 - 50 %, (42% in the case of SKOTAN *Yarrowia lipolytica* ).

The biomass obtained can be further processed, such as drying with well known techniques, particularly spray-drying, and then portioned and sold for use as a high-quality feed additive, particularly for bovine and poultry feeds.

## Claims

1. An industrial method of producing biomass, comprising culturing yeast of the SKOTAN strain of *Yarrowia lipolytica,* deposited in the IBPRS under the accession number KKP 2018 p., in a medium consisting of an aqueous solution containing from 20.0 to 70.0 g/l of a glycerol fraction resulting from biodiesel production, from 8.5 to 15.0 g/l (NH₄)₂SO_{4.}, from 1.5 to 6.5 g/l urea, from 0.5 to 3.0 g/l MgSO₄ x 7H₂O, from 0.1 to 2.0 g/l KH₂PO₄, from 0.1 to 2.0 g/l of yeast extract, at a temperature of 25 - 35°C, aeration at 0.2-4 L air/1L medium/minute, pH maintained at 2.5 to 7.5; up to the substantial exhaustion of glycerol in the medium, wherein the culture is in a periodic fashion comprising replacing a portion of the post-culture broth with a fresh portion of medium following each cycle,
wherein the culture is maintained in a volume of at least 1000 litres,
wherein biomass is produced at a rate from 1.5 to 3.0 g/Lh, and
wherein the dry biomass protein content is 30 to 50 % by mass.

2. A method according to Claim 1, **characterised in that** the separated post-culture broth yields from 15 to 35 g/L of yeast dry mass.

3. A method according to Claim 1, **characterised in that** the biomass production occurs with an overall efficiency of 0.4 to 0.5 g dry biomass/g glycerol fraction in the medium.

4. The SKOTAN strain of *Yarrowia lipolytica* deposited in the IBPRS under the accession number KKP 2018 p.

5. The use of the SKOTAN strain of *Yarrowia lipolytica* deposited in the IBPRS under the accession number KKP 2018 p in the reclamation of waste products of biodiesel production and thereby producing biomass.

6. A use according to Claim 5, **characterised in that** the biomass produced is used in feed production.

## Patentansprüche

1. Industrielles Verfahren zur Herstellung von Biomasse, umfassend Hefe des SKOTAN-Stammes von *Yarrowia lipolytica,* wie hinterlegt im IBPRS unter der Hinterlegungsnummer KKP 2018 p., in eine Medium bestehend aus einer wässrigen Lösung enthaltend von 20,0 bis 70,0 g/l einer Glycerinfraktion erhalten aus der Herstellung von Biodiesel, von 8,5 bis 15,0 g/l (NH₄)₂SO₄, von 1,5 bis 6,5 g/l Harnstoff, von 0,5 bis 3,0 g/l MgSO₄ x 7H₂O, von 0,1 bis 2,0 g/l KH₂PO₄, von 0,1 bis 2,0 g/l Hefeextrakt, bei einer Temperatur von 25 - 35°C, Belüftung von 0,2-4 L Luft/1L Medium/Minute, bei einem pH gehalten bei 2,5 bis 7,5; bis zur im wesentlichen Erschöpfung von Glycerin in dem Medium, wobei die Kultur auf eine periodische Weise durchgeführt wird, umfassend Ersetzen eines Teils des Post-Kulturmediums durch einem frischen Teil an Medium im Anschluss an jeden Zyklus,
wobei die Kultur bei einem Volumen von mindestens 1000 Liter gehalten wird,
wobei Biomasse bei einer Rate von 1,5 bis 3,0 g/Lh hergestellt wird, und
wobei der trockene Biomasse-Proteingehalt 30 bis 50 Masse% beträgt.

2. Verfahren nach Anspruch 1, **dadurch gekennzeichnet, dass** das abgetrennte Post-Kulturmedium von 15 bis 35 g/L an Hefe-Trockenmasse ergibt.

3. Verfahren nach Anspruch 1, **dadurch gekennzeichnet, dass** Biomasseherstellung mit einer Gesamteffizienz von 0,4 bis 0,5 g trockene Biomasse/g Glycerinfraktion in dem Medium auftritt.

4. SKOTAN-Stamm von *Yarrowia lipolytica,* wie hinterlegt im IBPRS unter der Hinterlegungsnummer KKP 2018 p.

5. Verwendung des SKOTAN-Stammes von *Yarrowia lipolytica,* wie hinterlegt im IBPRS unter der Hinteriegungsnummer KKP 2018 p in der Wiederverwendung von Abfallprodukten der Biodieselherstellung und der daraus resultierenden Produktion von Biomasse.

6. Verwendung nach Anspruch 5, **dadurch gekennzeichnet, dass** die hergestellte Biomasse in der Futtermittelproduktion verwendet wird.

## Revendications

1. Procédé industriel de production de biomasse, comprenant la culture d'une levure de la souche SKOTAN de *Yarrowia lipolytica,* déposée à l'IBPRS sous le numéro d'accession KKP 2018 p., dans un milieu constitué par une solution aqueuse contenant de 20,0 à 70,0 g/l d'une fraction glycérol résultant de la production d'un biodiesel, de 8,5 à 15,0 g/l de (NH₄)₂SO₄, de 1,5 à 6,5 g/l d'urée, de 0,5 à 3,0 g/l de MgSO₄ x 7H₂O, de 0,1 à 2,0 g/l de KH₂PO₄, de 0,1 à 2,0 g/l d'extrait de levure, à une température de 25-35°C, une aération à 0,2-4 l d'air/1 l de milieu/minute, un pH maintenu de 2,5 à 7,5 ; jusqu'à l'épuisement substantiel du glycérol dans le milieu, la culture se faisant de manière périodique comprenant le remplacement d'une partie du bouillon post-culture par une partie fraîche de milieu à l'issue de chaque cycle,
la culture étant maintenue dans un volume d'au moins 1000 litres,
la biomasse étant produite à une cadence de 1,5 à 3,0 g/l.h, et
la teneur en protéines de biomasse sèche allant de 30 à 50 % en masse.

2. Procédé selon la revendication 1, **caractérisé en ce que** le bouillon post-culture séparé a un rendement de 15 à 35 g/l de masse sèche de levure.

3. Procédé selon la revendication 1, **caractérisé en ce que** la production de biomasse a lieu avec une efficacité globale de 0,4 à 0,5 g de biomasse sèche/g de fraction glycérol dans le milieu.

4. Souche SKOTAN de *Yarrowia lipolytica* déposée à l'IBPRS sous le numéro d'accession KKP 2018 p.

5. Utilisation de la souche SKOTAN de *Yarrowia lipolytica* déposée à l'IBPRS sous le numéro d'accession KKP 2018 p dans la récupération de produits de déchets de production de biodiesel et produisant ainsi de la biomasse.

6. Utilisation selon la revendication 5, **caractérisée en ce que** la biomasse produite est utilisée dans la production de charges d'alimentation.
